(19)

Europäisches Patentamt

European Patent Office

Office européen des brevets

(11) **EP 4 179 972 A1**

(12) **EUROPEAN PATENT APPLICATION**

(43) Date of publication:
**17.05.2023 Bulletin 2023/20**

(21) Application number: **21208480.0**

(22) Date of filing: **16.11.2021**

(51) International Patent Classification (IPC):
**A61B 5/145** (2006.01)

(52) Cooperative Patent Classification (CPC):
**A61B 5/14532; A61B 5/1451; A61B 5/14517; A61B 5/14546**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **JOHNSON, Mark Thomas**
  **Eindhoven (NL)**

• **VAN LIESHOUT, Ron Martinus Laurentius**
  **Eindhoven (NL)**
• **HUIJBREGTS, Laurentia Johanna**
  **Eindhoven (NL)**
• **DELLIMORE, Kiran Hamilton J.**
  **Eindhoven (NL)**
• **PELSSERS, Eduard Gerard Marie**
  **Eindhoven (NL)**

(74) Representative: **Philips Intellectual Property & Standards**
**High Tech Campus 52**
**5656 AG Eindhoven (NL)**

(54) **ANALYTE DETERMINATION METHOD AND SENSING ASSEMBLY**

(57) Provided is a method (100) comprising providing (102) a nonblood bodily secretion sample having a concentration of an analyte. The analyte is separately detectable in blood. The method also comprises determining (104), independently of any measurement of said concentration, a measure of an absolute amount, in other words a total amount, of the analyte in the nonblood bodily secretion sample. Further provided is a sensing assembly for implementing the method, and a method for determining a correlation between such a measure of an absolute amount of the analyte in a nonblood bodily secretion sample and a blood concentration of the analyte in blood.

FIG. 7

EP 4 179 972 A1

**Description**

FIELD OF THE INVENTION

This invention relates to an analyte determination method wherein the analyte is in a nonblood bodily secretion sample, such as a sweat sample, and a sensing assembly for implementing the method using such a sample.
The invention further relates to a method of determining a correlation which can be used to determine a blood concentration of the analyte from a measure of the analyte determined using the nonblood bodily secretion sample.

BACKGROUND OF THE INVENTION

Non-invasive, semi-continuous and prolonged monitoring of biomarkers that indicate disease/health status and well-being is in demand for monitoring, for example, dehydration, stress, sleep, children's health and in perioperative monitoring.
Sweat, tear fluid and saliva may all be obtained non-invasively. Sweat is a particularly accessible biofluid, and is a rich source of information relating to the physiology and metabolism of a subject.
Some examples of clinically relevant components of sweat are $Na^+$, $Cl^-$ and/or $K^+$ to monitor dehydration, lactate as an early warning for inflammation (which is relevant to sepsis), glucose for diabetics and neonates, and cortisol in relation to sleep apnea and stress monitoring.
Continuous monitoring of high-risk patients, such as those with serious chronic conditions, pre- or post-operative patients, and the elderly, using sweat biomarker monitoring devices can provide higher quality diagnostic information than regular biomarker spot checks as normally done by repeatedly drawing multiple blood samples. Such continuous monitoring may be in a hospital setting or elsewhere. Human sweat alone or as mixture with sebum lipids may be an easily accessible source for biomarker measurements in wearable on-skin devices. For instance, cholesterol is an important biomarker associated with elevated risk in development of cardiovascular diseases. Inflammatory markers or cytokines, such as interleukins (e.g. TNF-a, IL-6) play an important role in the immune response and detection or disease monitoring of joint damage in rheumatoid and psoriatic arthritis, and bowel disease.
Examples of biomarkers that can be detected in eccrine/apocrine sweat using suitable capture species (antibodies, aptamers, molecular imprint polymers, etc.) are: small molecules such as urea, creatinine, cholesterol, triglycerides, steroid hormones (cortisol), glucose, melatonin; peptides and proteins, including cytokines such as IL-1alpha, IL-1beta, IL-6, TNF alpha, IL-8 and TGF-beta IL-6, Cysteine proteinases, DNAse I, lysozyme, Zn-$\alpha$2-glycoprotein, cysteine-rich secretory protein-3 and Dermcidin; and large biomarkers such as the Hepatitis C virus.
As summarized by Mena-Bravo and de Castro in "Sweat: A sample with limited present applications and promising future in metabolomics", J. Pharm. Biomed. Anal. 90, 139-147 (2014), frustrations with conventional sweat sensing techniques have been encountered in terms of the difficulty of producing enough sweat for analysis, the issue of sample evaporation, the lack of appropriate sampling devices, the need for trained staff, and issues relating to the normalization of the sampled volume. Moreover, it has been found that the results from sweat sensing can be highly variable, and a correlation between values determined from blood and sweat samples appears to be lacking for various biomarkers. In this respect, historical studies in this area have involved relatively crude sampling techniques, such as collecting large sweat volumes in bags or textiles. Deficiencies in such techniques may have been a contributing factor to this apparent lack of correlation.
Efforts have been made to address these issues by bringing wearable sensors into nearly immediate contact with sweat as it emerges from the skin. A recent example is the wearable patch presented by Gao et al. in "Fully integrated wearable sensor arrays for multiplexed in situ perspiration analysis", Nature 529, 509-514 (2016). The patch includes a sensor array for measuring $Na^+$, $K^+$, glucose, lactate, and skin temperature. However, the focus of this study is on the development and the integration of the sensors themselves which, whilst evidently crucial, does not address issues relating to sweat sample collection. The latter is mostly done by placing a several $cm^2$ sized absorbent pad between the skin and the sensor. The assumption is that, providing ample sweat is produced (hence tests are carried out on individuals that are exercising), the pad will absorb the sweat for analysis, and newly generated sweat will refill the pad and "rinse away" the old sweat. It is, however, likely that the time-dependent response of the sensor does not directly reflect the actual level of biomarkers over time because of accumulation effects. The sample collection and presentation to the published sensors may not be well-controlled so that continuous reliable sensing over a long period of time is difficult. Such patches may also not be designed to handle the tiny amounts of sweat that are produced under normal conditions, i.e. in the order of subnaloliters to nanoliters per minute per sweat gland.

SUMMARY OF THE INVENTION

The invention is defined by the independent claims. The dependent claims define advantageous embodiments.

According to an aspect there is provided a method comprising: providing a nonblood bodily secretion sample having a concentration of an analyte, said analyte being separately detectable in blood; and determining, independently of any measurement of said concentration, a measure of an absolute amount of the analyte in the nonblood bodily secretion sample.

The present disclosure is based on the insight that a more reliable correlation can be established between a blood concentration of the analyte in blood and the measure of the absolute amount of the analyte in the nonblood bodily secretion sample compared to the scenario in which a concentration of the analyte in the nonblood bodily secretion sample is used.

In particular, since the absolute amount of the analyte in the nonblood bodily secretion sample is determined independently of any measurement of the concentration of the analyte in the nonblood bodily secretion sample, there may be no requirement to measure the volume of the nonblood bodily secretion sample and derive an analyte concentration using this measured volume. The key is to make the analyte determination measurement independent of volume variation of the nonblood bodily secretion sample.

In some embodiments, the method further comprises calculating a measure of a blood concentration of the analyte in blood based on said determined measure of the absolute amount of the analyte in the nonblood bodily secretion sample.

The blood concentration of the analyte tends to be regarded as the clinical standard, and may be expressed, for example, in millimoles per liter (mM) or micromoles per liter ($\mu$M). The determined measure of the absolute amount of the analyte in the nonblood bodily secretion sample may be used as a proxy for the blood concentration of the analyte.

The calculating may comprise using a correlation between the measure of the blood concentration and the measure of the absolute amount. The correlation can, for instance, be in the form of a look-up-table, an analytic function or similar.

In at least some embodiments, the providing the nonblood bodily secretion sample comprises taking the nonblood bodily secretion sample from a subject's body over a given time period.

The given time period may be set according to the type of nonblood bodily secretion sample and the analyte. The given time period may be set such that the measure of the absolute amount of the analyte determined in the nonblood bodily secretion sample is reliably correlatable with the blood concentration of the analyte.

The given time period may be, for instance, in the range of 5 minutes to 24 hours, for example 5 minutes to 90 minutes, such as 10 to 60 minutes, e.g. about 15, about 30 or about 60 minutes.

In the case of sweat as the nonblood bodily secretion, a given time period in the above ranges may assist to spread out the above-mentioned time required for diffusion of the analyte/biomarker from blood through the interstitial fluid. This may assist to make this diffusion time effect negligible.

Any suitable total analysis technique can be used for determining the measure of the absolute amount of the analyte in the nonblood bodily secretion sample, independently of the concentration of the analyte in the nonblood bodily secretion sample.

The measure of the absolute amount of the analyte in the nonblood bodily secretion sample may, for instance, be a value expressed in grams or moles.

In some embodiments, the determining comprises contacting the nonblood bodily secretion sample with capture species configured to selectively interact and/or react with the analyte.

Alternatively or additionally, the determining may comprise using a single molecule assay to detect single molecules of the analyte.

The determination using such a single molecule assay, e.g. a single molecule immunoassay, may comprise counting the number of detected molecules of the analyte. In this way, a measurement of the absolute amount of the analyte can be made.

The capture species may be selected according to the analyte of interest. Selective interaction and/or reaction between capture species and a small molecule analyte, such as lactate, urea, creatinine and cortisol, can be achieved using, for example, an aptamer or a molecularly imprinted polymer as the capture species.

In some embodiments, the analyte is $Na^+$, $Cl^-$, $K^+$, $NH_4^+$, $H^+$, $Ca^{2+}$, lactate, ethanol, cortisol, glucose, urea, or creatinine. Analytes, in other words biomarkers, whose concentration is dependent on the secretion/excretion rate of the nonblood bodily secretion sample and/or for which the dependency of their concentration on the secretion/excretion rate is currently unknown are of particular interest, in other words $Na^+$, $Cl^-$, $H^+$, lactate, cortisol, urea, and $Ca^{2+}$ in the case of the nonblood bodily secretion being sweat.

Moreover, there are analytes/biomarkers that are measured in a specific timeframe in order to get a clinically relevant measurement. Examples are the kidney markers, such as urea, creatinine and $NH_4^+$.

Some further examples of clinically relevant components of sweat as the nonblood bodily secretion are $Na^+$, $Cl^-$ and/or $K^+$ to monitor dehydration, lactate as an early warning for inflammation (which is relevant to sepsis), glucose for diabetics and neonates, and cortisol in relation to sleep apnea and stress monitoring.

In at least some embodiments, the nonblood bodily secretion sample is a nonblood bodily fluid sample.

The nonblood bodily secretion sample may be a sweat sample. The nonblood bodily secretion sample can alternatively be a saliva sample or a tear fluid sample.

According to another aspect there is provided a sensing assembly comprising: a sampling stage configured to receive a nonblood bodily secretion sample having a concentration of an analyte, said analyte being separately detectable in blood; and an analysis stage configured to determine, independently of any measurement of said concentration, a measure of an absolute amount of the analyte in the nonblood bodily secretion sample.

In some embodiments, the sensing assembly further comprises a processing unit configured to calculate a measure of a blood concentration of the analyte in blood based on the determined absolute amount of the analyte in the nonblood bodily secretion sample.

The processing unit can, for example, be configured to calculate the measure of the blood concentration using a correlation established between the measure of the blood concentration of the analyte and the measure of the absolute amount.

In some embodiments, the sampling stage may be configured to receive the nonblood bodily secretion sample over a given time period. As described above in relation to the method, the given time period can be, for example, in the range of 5 minutes to 24 hours, for example 5 minutes to 90 minutes, such as 10 to 60 minutes, e.g. about 15, about 30 or about 60 minutes.

Alternatively or additionally, the sampling stage is arrangeable on or in a subject's body such as to receive the nonblood bodily secretion sample.

The sampling stage can, for instance, comprise a collecting member for collecting the nonblood bodily secretion sample over the given time period.

In a non-limiting example, the collecting member comprises an absorbent material for receiving and absorbing the nonblood bodily secretion sample from the area of the subject's body over the given time period.

In another non-limiting example, the collecting member comprises a membrane arranged such that the sample of the nonblood bodily secretion passes, e.g. is forced to pass, therethrough. The membrane may be activated to capture the analyte, which captured analyte may be available for subsequent determination of the absolute amount, e.g. at a later date. Such activation of the membrane can be implemented in any suitable manner, such as by the membrane comprising a molecularly imprinted polymer configured to selectively bind the analyte.

In some embodiments, the analysis stage comprises capture species configured to selectively interact and/or react with the analyte, with the capture species being arranged to contact the nonblood bodily secretion sample.

Alternatively or additionally, the analysis stage may comprise a plurality of discrete sensing areas, with each sensing area being arranged to detect a single molecule of the analyte.

In such an embodiment, the analysis stage may, for example, also include a counter arrangement configured to count the number of single molecules of the analyte detected by each of the discrete sensing areas, thereby to determine the measure of the absolute amount of the analyte in the nonblood bodily secretion sample.

As previously described in respect of the method, the analyte may be $Na^+$, $Cl^-$, $K^+$, $NH_4^+$, $H^+$, $Ca^{2+}$, lactate, ethanol, cortisol, glucose, urea, or creatinine.

Alternatively or additionally, the nonblood bodily secretion sample is a sweat sample. The nonblood bodily secretion can alternatively be saliva or tear fluid.

According to yet another aspect there is provided a wearable article comprising the sensing assembly according to any of the embodiments described above. The wearable article may, for example, further comprise a fastening element for fastening the sampling stage to the body of a subject. Such a fastening element may, for instance, comprise a suitable biocompatible adhesive and/or straps for attaching the sampling stage to the body of the subject.

According to a further aspect there is provided a method comprising: providing a plurality of blood samples; providing, for each of the plurality of blood samples, a nonblood bodily secretion sample taken from a subject at a time of taking one of the plurality of blood samples from the subject, wherein each of the nonblood bodily secretion samples has a concentration of an analyte; determining, for each of the non-blood bodily secretion samples, a measure of an absolute amount of the analyte in the respective nonblood bodily secretion sample independently of any measurement of said concentration in the respective nonblood bodily secretion sample; providing a measure of a blood concentration of the analyte in each of the blood samples; and determining a correlation between the measure of the absolute amount of the analyte and the blood concentration of the analyte using the determined measures of the absolute amount of the analyte in each of the nonblood bodily secretion samples and the determined measures of the blood concentration of the analyte in each of the blood samples.

Embodiments described herein in relation to the method of determining the correlation can be applicable to the sensing assembly and the method of determining the measure of the absolute amount of the analyte in the nonblood bodily secretion sample. Similarly embodiments described herein in relation to the sensing assembly and the method of determining the measure of the absolute amount of the analyte in the nonblood bodily secretion sample can be applicable to the method of determining the correlation.

BRIEF DESCRIPTION OF THE DRAWINGS

Embodiments of the invention are described in more detail and by way of non-limiting examples with reference to the accompanying drawings, wherein:

FIG. 1 provides a flowchart of a method according to an example;
FIG. 2 schematically depicts a sensing assembly according to an example;
FIG. 3 schematically depicts a sampling stage for collecting a nonblood bodily secretion sample;
FIG. 4 schematically depicts a sampling stage according to another example;
FIG. 5 schematically depicts core-shell particles in which the core of the particles comprises capture species;
FIG. 6 schematically depicts an analysis stage comprising a carrier having nanopores;
FIG. 7 provides a graph of absolute amount of lactate in a sweat sample vs concentration of lactate in blood; and
FIG. 8 provides a flowchart of a method of determining a correlation between a measure of the absolute amount of an analyte in a nonblood bodily secretion sample and a blood concentration of the analyte in blood.

DETAILED DESCRIPTION OF THE EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a method comprising providing a nonblood bodily secretion sample having a concentration of an analyte. The analyte is separately detectable in blood. The method also comprises determining, independently of any measurement of said concentration, a measure of an absolute amount, in other words a total amount, of the analyte in the nonblood bodily secretion sample. Further provided is a sensing assembly for implementing the method, and a method for determining a correlation between such a measure of an absolute amount of the analyte in a nonblood bodily secretion sample and a blood concentration of the analyte in blood.

In the case of analytes, in other words biomarkers, present both in blood and nonblood bodily secretions, it is desirable to use a measure of the analyte content of such a nonblood bodily secretion as a proxy for the blood concentration of the analyte.

The blood concentration of the analyte tends to be regarded as the clinical standard. However, nonblood bodily secretions, such as sweat, saliva and tear fluid, can be obtained less invasively than taking a blood sample. Alternatively or additionally, sampling such nonblood bodily secretions can facilitate prolonged monitoring of the subject, in particular outside of a healthcare setting.

A key challenge in utilizing the analyte content of such a nonblood bodily secretion as a proxy for the blood concentration of the analyte is that the concentration of the analyte in the nonblood bodily secretion may not reliably correlate with the blood concentration of the analyte.

For example, in the case of sweat as the nonblood bodily secretion, the concentration of certain biomarkers has been found to be dependent on the sweat rate. Selected biomarkers present in sweat and their currently understood dependency on sweat rate are presented in Table 1.

Table 1

| Biomarker | Concentration | Sweat rate dependency |
|---|---|---|
| $Na^+$ | 10-100 mM | |
| $Cl^-$ | 10-100 mM | Dependent |
| pH | 4.5-7 | |
| Lactate | 1-60 mM | |

(continued)

| Biomarker | Concentration | Sweat rate dependency |
|---|---|---|
| $K^+$ | 4-24 mM | None |
| $NH_4^+$ | 0.5-8 mM | |
| Ethanol | 2.5-22.5 mM | |
| Glucose | 0.2-0.6 mM | |
| Cortisol | 0.02-0.39 $\mu$M | Unknown |
| Urea | 2-6 mM | |
| $Ca^{2+}$ | 0.2-2.0 mM | |

In the case of biomarkers that can passively enter the sweat glands, dilution occurs at relatively high sweat rates due to the relatively large volume of sweat produced. Examples of such biomarkers are glucose, ethanol and cortisol. In the case of, for example, urea, a poor correlation with blood can be observed at relatively low sweat rates.

As sweat rates vary more rapidly than biomarkers and other molecules can enter into the sweat, all molecular concentrations in sweat are in principle affected by the above-mentioned dilution. In addition, for some molecules one or more additional factors can influence their concentration in sweat. Correlation between the concentration of the biomarker measured in sweat to the blood concentration of the biomarker may be further influenced by: (i) the reabsorption of biomarkers (e.g. $Na^+$/$Cl^-$) from sweat as the sweat leaves the sweat duct is a mechanism known to cause incorrect measurements of biomarker concentration, noting that such reabsorption may occur along the entire sweat duct from the sweat gland until the pore; (ii) production of the biomarker (e.g. lactate) by the gland cells producing the biomarker themselves; and (iii) the time taken for the biomarker to diffuse from blood via the interstitial fluid to the sweat glands. Regarding (iii), biomarkers may be reabsorbed from sweat, in particular by the movement, caused by filtration (see below), of the biomarkers from blood towards the interstitial fluid and the sweat glands.

The mass movement of fluids into and out of capillary beds, often referred to as bulk flow, is thought to involve two pressure-driven mechanisms: 1) volumes of fluid move from an area of higher pressure in a capillary bed to an area of lower pressure in the tissues via filtration, and 2) the movement of fluid from an area of higher pressure in the tissues into an area of lower pressure in the capillaries occurs via reabsorption. Two types of pressure interact to drive each of these movements: hydrostatic pressure and osmotic pressure.

The primary force driving fluid transport between the capillaries and tissues is hydrostatic pressure, which can be defined as the pressure of any fluid enclosed in a space. Blood hydrostatic pressure is the force exerted by the blood confined within blood vessels or heart chambers. Even more specifically, the pressure exerted by blood against the wall of a capillary is called capillary hydrostatic pressure (CHP), and is the same as capillary blood pressure. CHP is the force that drives fluid out of capillaries and into the tissues.

As fluid exits a capillary and moves into tissues, the hydrostatic pressure in the interstitial fluid correspondingly rises. This opposing hydrostatic pressure is called the interstitial fluid hydrostatic pressure (IFHP). Generally, the CHP originating from the arterial pathways is considerably higher than the IFHP, because lymphatic vessels are continually absorbing excess fluid from the tissues. Thus, fluid generally tends to move out of the capillary and into the interstitial fluid. This process is called filtration.

Filtration and reabsorption at the capillary occur in accordance with the balance of hydrostatic and osmotic pressures, as described in the Starling hypothesis:

$$Filtration\ force = k[(P_c + \pi_i) - (P_i + \pi_c)]$$

where k = permeability coefficient; $P_c$ = hydrostatic (blood) pressure in the capillary; $\pi_i$ = oncotic pressure in the interstitial fluid; Pi = hydrostatic pressure in the interstitial fluid; and $\pi_c$ = oncotic pressure in the capillary from plasma proteins. Note that the $P_c$ and Pi are "positive pressures" and $\pi_i$ and $\pi_c$ are "negative pressures" (generated by osmosis). Red cells remain in the bloodstream hence the term filtration but several biomolecules pass into the interstitial fluid.

It is also noted that certain biomarkers, such as small lipophilic (hydrophobic) analytes, such as steroid hormones (cortisol, testosterone, etc.) and hydrophilic drugs (methylxanthine, levodopa, ethanol, etc.), can exhibit a relatively strong correlation between sweat and blood concentrations. While such biomarkers are known to partition transcellularly through the lipophilic cell membranes, larger and/or more hydrophilic analytes, such as glucose, etc., are speculated to enter the sweat through a paracellular route, active channels or vesicular/exosomes, which may confound attempts to provide a sweat concentration-blood concentration correlation. In addition, hydrophilic drugs, such as penicillin, ibuprofen, and amoxicillin, are likely to be diluted.

In order to mitigate the above-identified problems, the present disclosure provides a method and apparatus in which a measure of an absolute amount of the analyte in the nonblood bodily secretion sample, e.g. a sweat sample, is determined, independently of any measurement of a concentration of the analyte in the nonblood bodily secretion sample.

A more reliable correlation can be established between a blood concentration of the analyte and the thus determined measure of the absolute amount of the analyte in the nonblood bodily secretion sample, e.g. collected in a given time period, compared to the scenario in which a concentration of the analyte in the nonblood bodily secretion sample is used.

In particular, since the absolute amount of the analyte in the nonblood bodily secretion sample is determined independently of any measurement of the concentration of the analyte in the nonblood bodily secretion sample, there may be no requirement to measure the volume of the nonblood bodily secretion sample and derive a biomarker concentration using this volume. The key is to make the analyte determination measurement independent of volume variation of the nonblood bodily secretion sample.

Taking a sweat sample as a representative example of the nonblood bodily secretion sample, it is noted that as the exercise load increases, the blood concentration of a given analyte may increase. However, at the same time the sweat analyte concentration may decrease (due to the above-described dilution by the increased volume of sweat produced). However, as the exercise load increases, the volume of sweat produced also increases, and this volume increase can exceed the reduction in sweat analyte concentration. As a consequence, the absolute, in other words total, amount of the analyte in the sweat sample (i.e., volume multiplied by concentration) can also increase with exercise load, and as such can correlate with the blood concentration, noting that the blood volume remains constant.

It is also noted, in the case of such a sweat sample, that the volume of sweat relates to the number of active sweat glands, the sweat rate per gland and the time period over which the glands are active (i.e., volume = sweat rate x number of active glands x time of activation).

In at least some embodiments, the measure of the absolute amount of the analyte in the nonblood bodily secretion sample taken from a particular reference body area, e.g. area of the skin in the case of sweat sampling, during a given time period is determined.

In some embodiments, as will be described in more detail herein below, such a measure of the absolute amount of the analyte in the nonblood bodily secretion sample then be compared or correlated with a blood concentration of the analyte.

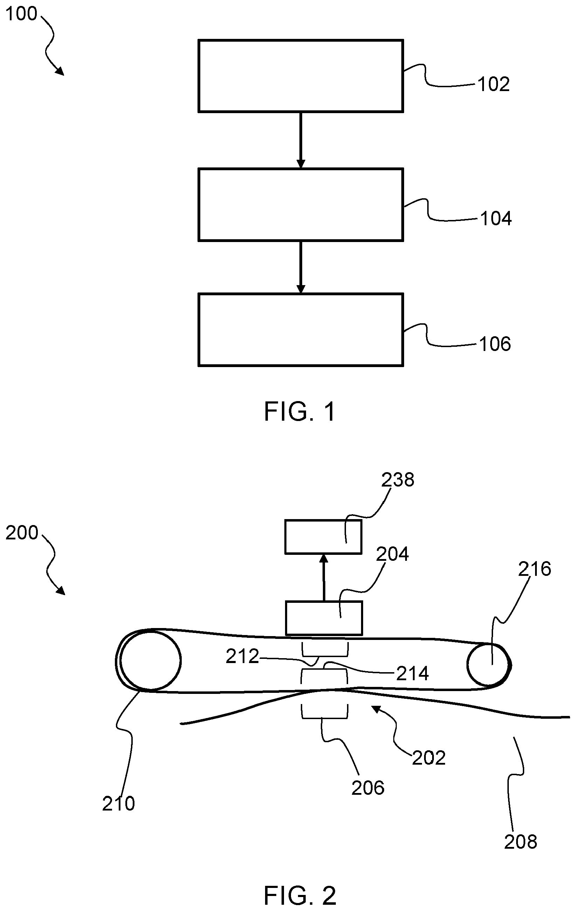

FIG. 1 provides a flowchart of a method 100 according to an example. The method 100 comprises providing 102 a nonblood bodily secretion sample having a concentration of an analyte. The method 100 further comprises determining 104, independently of any measurement of said concentration, a measure of an absolute amount of the analyte in the nonblood bodily secretion sample.

The nonblood bodily secretion can be any suitable nonblood bodily secretion, provided that the analyte potentially included in the nonblood bodily secretion sample is also detectable in the subject's blood. Thus, the measure of the absolute amount of the analyte in the nonblood bodily secretion can be used as a proxy for the blood concentration of the analyte in blood.

In some embodiments, the nonblood bodily secretion is sweat, saliva, or tear fluid. Particular mention is made of sweat owing to the accessibility of this biofluid, and the relevance of the analytes contained in sweat to the physiology and metabolism of a subject.

The analyte can be, for example, $Na^+$, $Cl^-$, $K^+$, $NH_4^+$, $H^+$, $Ca^{2+}$, lactate, ethanol, cortisol, glucose, urea, and creatinine.

Since the key is to make the analyte determination measurement independent of volume variation of the nonblood bodily secretion sample, the analytes/biomarkers listed, for instance, in Table 1 whose concentration is secretion/excretion rate-dependent and/or for which the dependency of their concentration on secretion/excretion rate is currently unknown are of particular interest, in other words $Na^+$, $Cl^-$, $H^+$, lactate, cortisol, urea, and $Ca^{2+}$.

Moreover, there are analytes/biomarkers that are measured in a specific timeframe in order to get a clinically relevant measurement. Examples are the kidney markers, such as urea and creatinine.

In a non-limiting example, an absolute amount determination of creatinine, urea and/or $NH4^+$ in a sweat sample could replace determination of the same waste products in urine, e.g. monitored over a 24 hour period.

Some further examples of clinically relevant components of sweat as the nonblood bodily secretion are $Na^+$, $Cl^-$ and/or $K^+$ to monitor dehydration, lactate as an early warning for inflammation (which is relevant to sepsis), glucose for diabetics and neonates, and cortisol in relation to sleep apnea and stress monitoring.

FIG. 2 schematically depicts a sensing assembly 200 according to an example. The sensing assembly 200 comprises a sampling stage 202 configured to receive the nonblood bodily secretion sample, and an analysis stage 204 configured to determine, independently of any measurement of the concentration of the analyte in the nonblood bodily secretion sample, the measure of the absolute amount of the analyte in the nonblood bodily secretion sample.

In at least some embodiments, the providing 102 comprises taking, in other words collecting, the nonblood bodily secretion sample from an area of a subject's body, in other words a reference area, over a given time period.

The given time period may be set according to the type of nonblood bodily secretion sample and the analyte. The time may be set such that the measure of the absolute amount of the analyte determined in the nonblood bodily secretion sample is reliably correlatable with the blood concentration of the analyte.

The given time period may be, for instance, in the range of 5 minutes to 24 hours, for example 5 minutes to 90 minutes, such as 10 to 60 minutes, e.g. about 15, about 30 or about 60 minutes.

In the case of sweat as the nonblood bodily secretion, such a given time period may assist to spread out the above-mentioned time required for diffusion of the analyte/biomarker from blood through the interstitial fluid, and therefore may assist to make this diffusion time effect negligible.

Referring to FIG. 2, the sampling stage 202 may be arrangeable on or in a subject's body such as to receive the nonblood bodily secretion sample from the area 206 of the subject's body 208 over the given time period.

In examples in which the nonblood bodily secretion is sweat, the sampling stage 202 may be arrangeable on or proximal to the skin of the subject in order to collect the sweat sample therefrom.

More generally, the sensing assembly 200 may be included in a wearable article. The wearable article may, for example, further comprise a fastening element (not shown) for fastening the sampling stage to the body of a subject. Such a fastening element may, for instance, comprise a suitable biocompatible adhesive and/or straps for attaching the sampling stage to the body of the subject.

In some embodiments, the providing 102 comprises collecting the nonblood bodily secretion sample using a collecting member 210. Such a collecting member 210 can, for example, be included in the sampling stage 202 of the sensing assembly 200.

In some embodiments, the collecting member 210 comprises an absorbent material 212 for receiving and absorbing the nonblood bodily secretion sample from the area 206 of the subject's body 208 over the given time period. The measure of the absolute amount of the analyte in the thus collected nonblood bodily secretion sample can then be determined 104, as will be described in more detail herein below.

In some embodiments, such as that shown in FIG. 2, the collecting member 210 comprises the absorbent material 212 and a further absorbent material 214 for receiving and absorbing a further nonblood bodily secretion sample over a further given time period. The collecting member 210 may be configured such that the absorbent material 212 in which the nonblood bodily secretion sample is collected is replaced by the further absorbent material 214 such that the further absorbent material 214 can receive and absorb the further nonblood bodily secretion sample.

More generally, the sampling stage 202 may comprise a mechanism 216, e.g. comprising a rotatable drum, configured to move the absorbent material 212 away from the area 206 of the body 208 from which the nonblood bodily secretion sample is collected once the given time period has elapsed.

In some embodiments, the mechanism 216 may be configured to move the absorbent material 212 away from the area 206 of the body 208 from which the nonblood bodily secretion is collected to the analysis stage 204 at which the measure of the absolute amount of the analyte in the nonblood bodily secretion sample is determined 104.

In the non-limiting example shown in FIG. 2, the mechanism 216 can, as well as moving the absorbent material 212 away from the area 206 of the body 208, bring the above-mentioned further absorbent material 214 to the area 206 of the body 208 vacated by the absorbent material 212.

In embodiments in which the collecting member 210 comprises the absorbent material 212 and the further absorbent material 214, the determination 104 of the absolute amount of the analyte in the nonblood bodily secretion sample collected in the absorbent material 212 may be implemented while the further absorbent material 214 is collecting the further nonblood bodily secretion sample. An example of this is shown in FIG. 2.

In a specific non-limiting example, the collecting member 210 comprises filter paper as the absorbent material and the further absorbent material. In such an example, a portion of the filter paper corresponding to the above-described absorbent material 212 may collect the nonblood bodily secretion, e.g. sweat, sample for the given time period. The portion of the filter paper may subsequently be moved, e.g. via the above-described mechanism 216, to the analysis stage 204 while a further portion of the filter paper corresponding to the above-described further absorbent material 214 is collecting the further nonblood bodily secretion, e.g. sweat, sample.

The portions of filter paper may, for example, be discrete, in other words separate from each other. In other examples, the portions of filter paper may be included in a continuous roll, from which fresh portions of the filter paper can be continuously exposed to the area 206 of the body 208 from which the nonblood bodily secretion is collected.

In some embodiments, the collecting member 210 comprises a membrane arranged such that the sample of the nonblood bodily secretion, e.g. sweat, passes, e.g. is forced to pass, therethrough.

The membrane may be activated to capture the analyte, which captured analyte may be available for subsequent determination 104 of the absolute amount, e.g. at a later date. Such activation of the membrane can be implemented in any suitable manner, such as by the membrane comprising a molecularly imprinted polymer configured to selectively bind the analyte.

Molecularly imprinted polymers are known, for example, for the selective binding of lactate and cortisol. Reference is made to, for example, Alizadeh et al., Talanta 192 (2019) 103-111, and Parlak et al., Sci. Adv. 2018; 4 eaar2904.

The determining 104, independently of any measurement of the concentration of the analyte in the nonblood bodily secretion sample, the measure of the absolute amount of the analyte in the nonblood bodily secretion sample can be implemented in any suitable manner, such as by contacting the nonblood bodily secretion sample with capture species configured to selectively interact and/or react with the analyte.

A typical determination 104 measurement of component A with a capture species B can be described as A + B → AB. Such a measurement can be rendered independent of the volume of nonblood bodily secretion sample by: a) making the reaction to AB dominant, for example by increasing the amount of capture species and/or by modifying the capture species to more tightly bind the analyte, or by shifting AB, once formed, away from A + B. An alternative approach b) is to minimize or prevent the reversion of AB to A + B, for example by using a size, conformation and/or charge change occurring from A to AB.

More generally, the capture species may be selected according to the analyte of interest. Selective interaction and/or reaction between capture species and a small molecule analyte, such as lactate, urea, creatinine and cortisol, can be achieved using, for example, an aptamer or a molecularly imprinted polymer as the capture species.

It is further noted that antibodies are known which can act as capture species for binding cortisol. Reference is made to, for example, Torrente-Rodriguez et al., Matter 2, 921-937, 2020.

In some non-limiting examples, the capture species may be, or comprise, an enzyme. Such an enzyme may selectively bind the analyte, and in some examples convert the analyte in a chemical reaction. This binding, and in some examples reaction, can be used in the determination 104. For example, lactate can be selectively bound and converted by lactate dehydrogenase.

In the case of the analyte being an ion, the capture species may be in the form of an ion selective membrane, e.g. ion selective membranes integrated in ion selective electrodes.

In some non-limiting examples, a change of charge due to binding of a particular ion, e.g. a sodium ion, may cause the conformation and/or charge of the capture species to change. This change can then be used to force the A + B → AB equilibrium to the AB side.

In some embodiments, the analysis stage 204 of the sensing assembly 200 may comprise a sensing element comprising the capture species.

In embodiments in which the capture species are employed in the determination 104, the entirety, or substantially all, of the nonblood bodily secretion, e.g. sweat, sample may be provided to the sensing element of the analysis stage 204.

The sensing element may, for example, comprise a capture surface on which the capture species are provided, e.g. immobilized, and over which the nonblood bodily secretion sample either flows, or in some examples over which the nonblood bodily secretion sample circulates, until all, or substantially all, the analyte is removed from the nonblood bodily secretion sample and is captured by the capture species.

The amount of biomarker can be ascertained by any known method for measuring interaction and/or reaction of the analyte with the capture surface, such as electrical, optical, and/or magnetic measurement approaches.

It is noted that in order to make repeated determinations 104, it may be necessary for a significant excess of capture species to be provided on the capture surface, or for the capture surface to be subjected to a replenishing process. An example of such a replenishing process is described in WO 2020/099570 A1.

Alternatively, the entirety, or substantially all, of the analyte, is collected by the collecting member 210, e.g. by the above-described membrane, before the absolute amount of the thus captured analyte is determined 104 at the analysis stage 204.

In such embodiments, the analysis stage 204 can use any suitable analysis principle for determining the absolute amount of the analyte. The determination 104 can be made, for instance, following liberation of the analyte from the capture species.

For example a chromatographic technique can be used in which elution of the analyte, or in some examples the analyte still bound to the capture species, through a chromatographic column included in the analysis stage 204 is monitored by a suitable detector, and the height or integral of the detected signal corresponding to the analyte (or analyte bound to the capture species) is used to derive the measure of the absolute amount of the analyte.

The chromatographic column may comprise one or more phases suitable for separating the analyte, or in some examples the analyte still bound to the capture species, from other components which may be present in the nonblood bodily secretion sample.

An internal standard can, for example, be used to assist in the quantification of the analyte, e.g. using the ratio of the area under the analyte signal and the area under the signal corresponding to the internal standard.

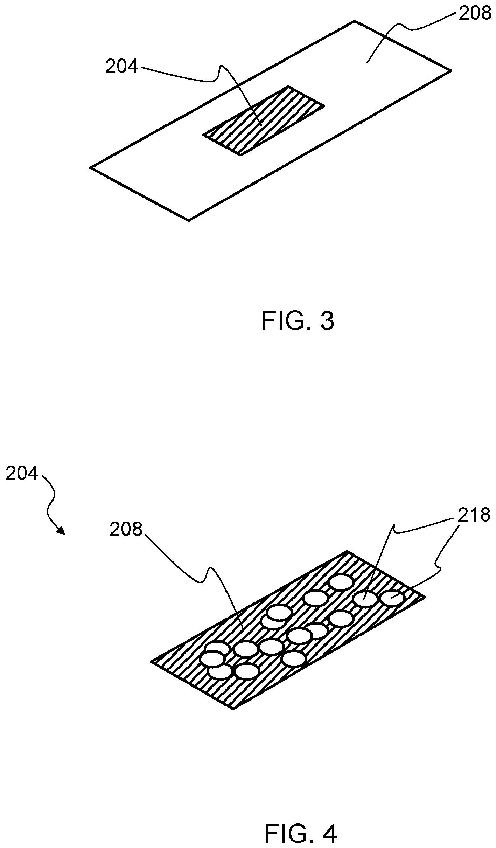

FIG. 3 shows an exemplary sampling stage 204 in the form of a wearable patch arranged on the body 208, in this case the skin. Thus, the sampling stage 204 shown in FIG. 3 can be used to collect sweat as the nonblood bodily secretion.

In some embodiments, such as in the non-limiting example depicted in FIG. 4, the analyte is captured *in situ,* for example in a fluidic system included in the sampling stage 202, at the area 206 of the body 208 at which the nonblood bodily secretion sample is collected, or in the vicinity of the sweat glands, using core-shell hydrogel particles 218. Such core-shell hydrogel particles 218 may comprise the capture species in the core of the particles surrounded by a hydrogel shell.

The core-shell hydrogel particles 218 may be brought into contact with the nonblood bodily secretion sample, and, after a fixed amount of time, can be transported to the analysis stage 204 at which the determination 104 may be made.

For example, at the analysis stage 204, the analyte, or the analyte bound to the capture species, e.g. antigen, can be removed, e.g. by elution, from the core-shell particles 218 and measured in a fixed volume.

Returning to FIG. 4, the exemplary sampling stage 204, e.g. in the form of the wearable patch described above in relation to FIG. 3, comprises the collecting member 210, with the core-shell particles 218 provided in the collecting member 210. The collecting member 210 in this example may comprise, or be in the form of, the above-described absorbent material 212.

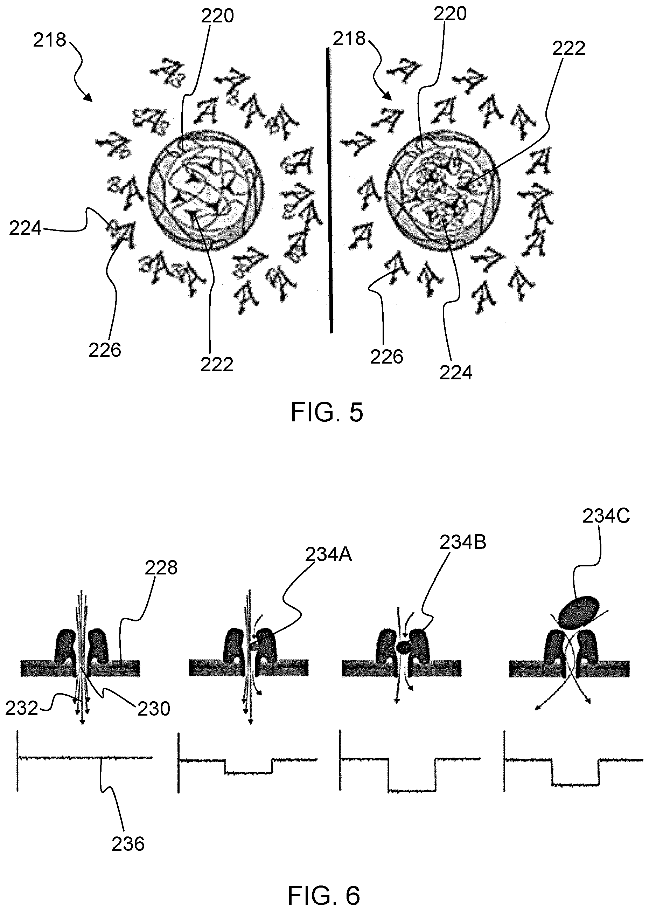

The schematic depiction of the core-shell particles 218 provided in FIG. 5 shows the hydrogel shell 220, and the capture species 222 in the core of the core-shell particles 218. Moreover, the left hand pane of FIG. 5 schematically depicts contacting the core-shell particles 218 with the analyte 224. In this particular example, the analyte 224 is associated with albumin 226, with the analyte 224 and the albumin 226 being included in the nonblood bodily secretion sample in this case.

The right hand pane on FIG. 5 shows all of the analyte 224 being bound by the capture species 220 in the core of the core-shell particles 218, leaving the albumin 226 without the analyte 224 on the outside the core-shell particles 218.

In some embodiments, an indicator approach is used in the determination 104 of the measure of the absolute amount of the analyte in the nonblood bodily secretion sample. Such an indicator approach may also require that the entirety, or substantially all, of the nonblood bodily secretion, e.g. sweat, sample is provided to the sensing element of the analysis stage 204, or the entirety, or substantially all, of the analyte, is collected by the collecting member 210, e.g. the by above-described membrane, before the absolute amount of the thus captured analyte is determined 104 at the analysis stage 204.

In such embodiments, the determination 104 may comprise contacting an indicator material, e.g. indicator liquid, configured to selectively interact and/or react with the analyte with the nonblood bodily secretion, e.g. sweat, sample after the given time period during which the nonblood bodily secretion sample is collected. The absolute amount of the analyte can be determined 104 by any of the known methods for measuring the total reaction and/or interaction between the indicator material and the analyte, such as electrical, optical or magnetic measurement approaches.

For example, reaction and/or interaction of the analyte with the indicator material may result in the indicator material, e.g. indicator liquid, changing color such as to enable the measure of the absolute amount of the analyte in the nonblood bodily secretion sample to be determinable 104.

Another approach is to determine the measure of the absolute amount of the analyte in the nonblood bodily secretion sample by measuring the total optical activity in a fluid mixture comprising the indicator material and the analyte in the nonblood bodily secretion sample following the given time period.

It is noted that in order to make repeated determinations 104, it may be necessary for a significant excess of the indicator material to be provided relative to the analyte, or for the indicator material to be replenished, such as by flushing the sampling volume and re-starting the sampling of the nonblood bodily secretion.

In some embodiments, such as that shown in FIG. 6, the analysis stage 204 comprises a carrier 228 having pores 230, in particular nanopores 230. A detectable current flow 232 can be provided through the pores 230. In such embodiments, the analysis stage 204 comprises a detector (not shown) configured to detect the current flow 232.

When the current flow 232 is interrupted (for example by a molecule 234A, 234B, 234C) this can be measured as a drop in the signal 236, as shown in FIG. 6.

Nanopores 230 were first developed for DNA sequencing and later also adapted to detect other molecules. The detection of specific analytes, and in particular molecular analytes, can either be done by i) size exclusion, where the pores 230 are such that only the molecule of interest can flow through and/or create a specific signal, ii) adding a transporting protein that only transports/recognizes the molecule of interest, or iii) by adding a capture species to the pore 230 that induces an increased residence time.

In each of these cases, the current flow may be one-way and therefore the pores 230 essentially count the total amount of analyte molecules pulled through the pores 230. An electrical current can be used to prevent any backflow of the analyte and/or a protein can be used to prevent such backflow.

More generally, the determination 104 of the measure of the absolute amount of the analyte in the nonblood bodily secretion sample may employ a counting method, such as using nanopores 230 configured to count each time each analyte molecule or ion is transported or a chemiluminescence involving counting the number of photons associated with each analyte molecule or ion. In such examples, the analyte molecules/ions may be removed after the measurement.

The determination 104 of the measure of the absolute amount of the analyte in the nonblood bodily secretion sample can, in some embodiments, be implemented using a single molecule assay, e.g. a single molecule immunoassay.

In such a single molecule assay (in other words a so-called "digital assay" approach), a plurality of discrete sensing areas are included in the analysis stage 204, with each discrete sensing area, e.g. detection well, being arranged to detect a single molecule of the analyte. In such an embodiment, the analysis stage 204 may include a counter arrangement configured to count the number of single molecules of the analyte detected by each of the discrete sensing areas thereby to determine the measure of the absolute amount of the analyte in the nonblood bodily secretion sample.

In other words, the determination 104 using the single molecule assay may comprise counting the number of detected molecules of the analyte. In this way, a measurement of the absolute amount of the analyte can be made.

It is noted that such discretized sensing areas can detect fewer than one analyte molecule of interest when considered on a statistical/distribution level.

An example of a single molecule assay is described by Rissin et al. in "Multiplexed single molecule immunoassays", Lab Chip. 2013, 13(15):2902-11.

With respect to the measurements, various known examples can be integrated with the methods according to the present disclosure.

For example, nanoparticles (which can, for example, be simply referred to as "beads") can be used to force the molecules into the above-described detection wells, thereby providing a nano-well approach.

For example, nanoparticles can be used to detect binding events. When nanoparticles in an area with zero or one molecule they may either exhibit Brownian motion interaction when no molecule is present or a specific immobility status when one molecule is present. This can be detected optically and enhanced with magnetic interaction.

An example of this is described by Ranzoni et al. in "Frequency-Selective Rotation of Two-Particle Nanoactuators for Rapid and Sensitive Detection of Biomolecules", Nano Lett. 2011, 11, 5, 2017-2022.

Another example of a single molecule assay is a proximity ligation assay (PLA). Such a PLA may only generate a signal when a molecule is present.

In yet another example, a conventional enzyme-linked immunosorbent-type assay can be used, subject to, for example, provision of suitable wash and/or separation steps upstream of the analysis stage 204.

In at least some embodiments, the method 100 further comprises calculating 106 a measure of a blood concentration of the analyte in blood based on the determined measure of the absolute amount of the analyte in the nonblood bodily secretion sample.

In at least some embodiments, the calculating 106 comprises using a correlation established between the measure of the blood concentration and the measure of the absolute amount.

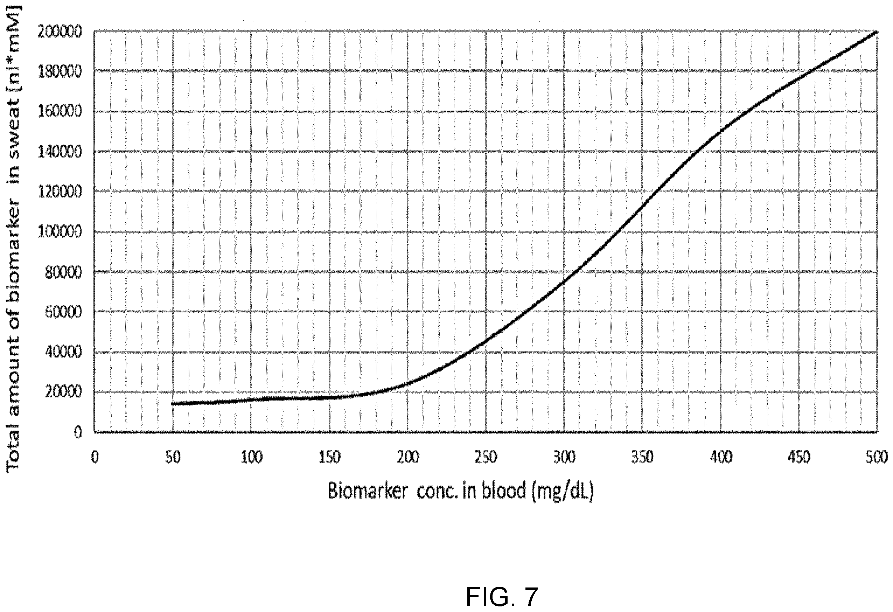

Such a correlation is shown in graphical form in FIG. 7 for the specific non-limiting example of the absolute amount of lactate in a sweat sample vs the blood concentration of lactate.

The correlation can, for instance, be in the form of a look-up-table, an analytic function or similar.

In some embodiments, the sensing assembly 200 comprises a processing unit 238 configured to calculate the measure of the blood concentration of the analyte in blood based on the determined measure of the absolute amount of the analyte in the nonblood bodily secretion sample.

The processing unit 238 can be configured to calculate the measure of the blood concentration using the above-described correlation between the measure of the blood concentration and the measure of the absolute amount. The correlation can, for instance, be in the form of a look-up-table, an analytic function or similar, as previously described.

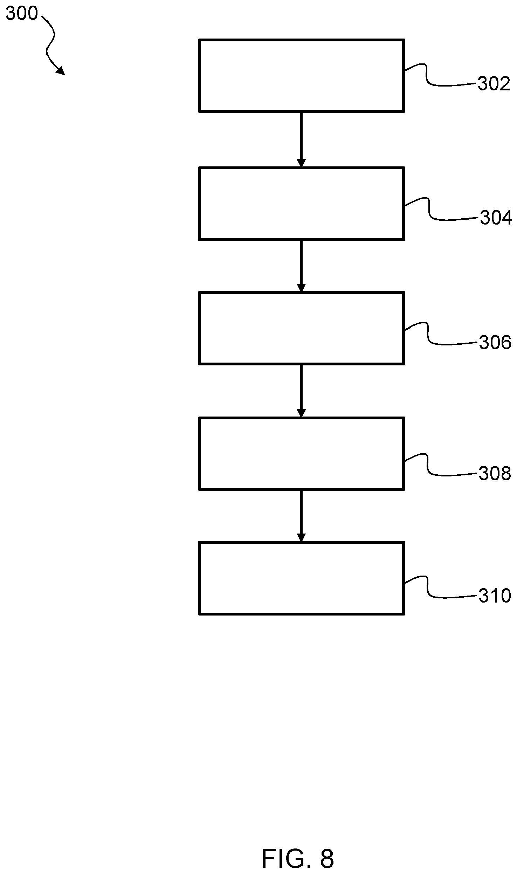

Further provided is a method 300 for providing the above-described correlation, as shown in FIG. 8. The method 300 comprises providing 302 a plurality of blood samples, and providing 304, for each of the plurality of blood samples, a nonblood bodily secretion sample taken from a subject at a time of taking one of the plurality of blood samples from the subject.

The method 300 further comprises determining 306, for each of the non-blood bodily secretion samples, the measure of the absolute amount of the analyte in the respective nonblood bodily secretion sample independently of any measurement of the concentration of the analyte in the respective nonblood bodily secretion sample. The determining 306 may be implemented according to any of the examples described above in respect of step 104 in FIG. 1, and the analysis stage 204 in FIG. 2.

The method 300 further comprises providing 308, e.g. determining, a measure of a blood concentration of the analyte in each of the blood samples. This may utilize any known technique for determining the blood concentration of the analyte.

In step 310, the correlation between the measure of the absolute amount of the analyte in the nonblood bodily secretion sample and the blood concentration of the analyte is determined using the determined measures of the absolute amount of the analyte in each of the nonblood bodily secretion samples and the determined measures of the blood concentration of the analyte in each of the blood samples.

In this way, a calibration curve of the type shown in FIG. 7 can, for example, be provided.

The apparatus, systems and methods of the present disclosure may be applied for non-invasive, semi-continuous and prolonged monitoring of biomarkers that indicate health and well-being, for example for monitoring dehydration, stress, sleep, children's health and in perioperative monitoring. As well as being applicable for subject monitoring in general, the present apparatus, systems and methods may be specifically applied to provide an early warning for sudden deterioration of patients in the General Ward and Intensive Care Unit, or for investigation of sleep disorders. Currently, measurements may only be made in a spot-check fashion when a patient is visiting a doctor, although it is noted that the present disclosure may also be usefully applied in performing such spot-check measurements.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. Measures recited in mutually different dependent claims can advantageously be combined. Any reference signs in the claims should not be construed as limiting the scope.

**Claims**

1. A method (100) comprising:

   providing (102) a nonblood bodily secretion sample having a concentration of an analyte, said analyte being separately detectable in blood; and
   determining (104), independently of any measurement of said concentration, a measure of an absolute amount of the analyte in the nonblood bodily secretion sample.

2. The method (100) according to claim 1, further comprising:
   calculating (106) a measure of a blood concentration of the analyte in blood based on said determined measure of the absolute amount of the analyte in the nonblood bodily secretion sample.

3. The method (100) according to claim 2, wherein said calculating (106) comprises using a correlation between the measure of the blood concentration and the measure of the absolute amount.

4. The method (100) according to any of claims 1 to 3, wherein the providing (102) comprises taking the nonblood bodily secretion sample from a subject's body over a given time period.

5. The method (100) according to any of claims 1 to 4, wherein the determining (104) comprises contacting the nonblood bodily secretion sample with capture species configured to selectively interact and/or react with the analyte.

6. The method (100) according to any of claims 1 to 5, wherein the determining (104) comprises using a single molecule assay to detect single molecules of the analyte.

7. The method (100) according to any of claims 1 to 6, wherein the analyte is $Na^+$, $Cl^-$, $K^+$, $NH_4^+$, $H^+$, $Ca^{2+}$, lactate, ethanol, cortisol, glucose, urea, or creatinine.

8. The method (100) according to any of claims 1 to 7, wherein the nonblood bodily secretion sample is a sweat sample.

9. A sensing assembly (200) comprising:

   a sampling stage (202) configured to receive a nonblood bodily secretion sample having a concentration of an analyte, said analyte being separately detectable in blood; and
   an analysis stage (204) configured to determine, independently of any measurement of said concentration, a measure of an absolute amount of the analyte in the nonblood bodily secretion sample.

10. The sensing assembly (200) according to claim 9, comprising a processing unit (206) configured to calculate a measure of a blood concentration of the analyte in blood based on said determined absolute amount of the analyte in the nonblood bodily secretion sample; optionally wherein the processing unit is configured to calculate said measure of the blood concentration of the analyte using a correlation between the measure of the blood concentration and the measure of the absolute amount.

11. The sensing assembly (200) according to claim 9 or claim 10, wherein the sampling stage (202) is configured to receive the nonblood bodily secretion sample over a given time period; and/or wherein the sampling stage is arrangeable on or in a subject's body such as to receive the nonblood bodily secretion sample.

**12.** The sensing assembly (200) according to any of claims 9 to 11, wherein the analysis stage (204) comprises an excess of capture species configured to selectively interact and/or react with the analyte, said excess of capture species being arranged to contact the nonblood bodily secretion sample.

**13.** The sensing assembly (200) according to any of claims 9 to 12, wherein the analyte is $Na^+$, $Cl^-$, $K^+$, $NH_4^+$, $H^+$, $Ca^{2+}$, lactate, ethanol, cortisol, glucose, urea, or creatinine; and/or wherein the nonblood bodily secretion is sweat.

**14.** A wearable article comprising the sensing assembly (200) according to any of claims 9 to 13.

**15.** A method (300) comprising:

providing (302) a plurality of blood samples;
providing (304), for each of the plurality of blood samples, a nonblood bodily secretion sample taken from a subject at a time of taking one of the plurality of blood samples from the subject, wherein each of the nonblood bodily secretion samples has a concentration of an analyte;
determining (306), for each of the non-blood bodily secretion samples, a measure of an absolute amount of the analyte in the respective nonblood bodily secretion sample independently of any measurement of said concentration of the analyte in the respective nonblood bodily secretion sample;
providing (308) a measure of a blood concentration of the analyte in each of the blood samples; and
determining (310) a correlation between the measure of the absolute amount of the analyte and the blood concentration of the analyte using the determined measures of the absolute amount of the analyte in each of the nonblood bodily secretion samples and the determined measures of the blood concentration of the analyte in each of the blood samples.

100

102

104

106

FIG. 1

238

200

204

216

212

214

210

206

202

208

FIG. 2

FIG. 3

FIG. 4

FIG. 5

FIG. 6

FIG. 7

FIG. 8

Europäisches
Patentamt

European
Patent Office

Office européen
des brevets

## EUROPEAN SEARCH REPORT

Application Number

EP 21 20 8480

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/045247 A1 (VIVOMEDICAL INC [US]; POTTS RUSSELL O [US]; MOYER JAMES W [US]) 22 April 2010 (2010-04-22) | 1-5,7-15 | INV. A61B5/145 |
| Y | * paragraphs [0058], [0094] – [0099]; figures 1,3b * | 6 | |
| Y | WANG XU ET AL: "Competitive Immunoassays for the Detection of Small Molecules Using Single Molecule Arrays", JOURNAL OF THE AMERICAN CHEMICAL SOCIETY, vol. 140, no. 51, 29 November 2018 (2018-11-29), pages 18132-18139, XP055914817, ISSN: 0002-7863, DOI: 10.1021/jacs.8b11185 * the whole document * | 6 | |

TECHNICAL FIELDS
SEARCHED      (IPC)

A61B

The present search report has been drawn up for all claims

| Place of search | Date of completion of the search | Examiner |
|---|---|---|
| The Hague | 22 April 2022 | Lommel, André |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 20 8480

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2022

| Patent document cited in search report | Publication date | Patent family member(s) | Publication date |
|---|---|---|---|
| WO 2010045247 A1 | 22-04-2010 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020099570 A1

### Non-patent literature cited in the description

- **MENA-BRAVO ; DE CASTRO.** Sweat: A sample with limited present applications and promising future in metabolomics. *J. Pharm. Biomed. Anal.,* 2014, vol. 90, 139-147
- **GAO et al.** Fully integrated wearable sensor arrays for multiplexed in situ perspiration analysis. *Nature,* 2016, vol. 529, 509-514
- **ALIZADEH et al.** *Talanta,* 2019, vol. 192, 103-111
- **PARLAK et al.** *Sci. Adv.,* 2018, vol. 4, 2904
- **TORRENTE-RODRIGUEZ et al.** *Matter,* 2020, vol. 2, 921-937
- **RISSIN et al.** Multiplexed single molecule immunoassays. *Lab Chip,* 2013, vol. 13 (15), 2902-11
- **RANZONI et al.** Frequency-Selective Rotation of Two-Particle Nanoactuators for Rapid and Sensitive Detection of Biomolecules. *Nano Lett,* 2011, vol. 11 (5), 2017-2022